# EUROPEAN PATENT APPLICATION

(11) **EP 2 082 768 A1**
(43) Date of publication of application: **29.07.2009**
(21) Application number: 08100884.9
(22) Date of filing: 24.01.2008
(51) Int. Cl.: A61M 15/00

(54) **Inhaler**

(71) Applicant: Vectura Delivery Devices Limited, Chippenham, Wiltshire SN14 6FH (GB)
(72) Inventor: Campling, Nicholas John, Peterborough PE3 8LB (GB); Biddle, Howard William, Cambridge CB24 9JJ (GB); Bradley, Duncan James, Surrey GU6 7QJ (GB); Sarkar, Matthew, Cambridge CB4 3JU (GB)
(74) Representative: Grey, Ian Michael

(57) **Abstract**

An inhaler is disclosed. It comprises a housing to receive a strip of sealed blisters each containing a dose of medicament and means to move a strip received within the housing such that each blister is sequentially aligned with a blister opening member for opening said aligned blister to facilitate inhalation of said dose. The inhaler also comprises means for re-sealing opened blisters previously aligned with the blister opening member by applying adhesive tape to the strip.

## Description

The present invention relates to an inhalation device for oral or nasal delivery of medicament in powdered form and to an inhaler containing a strip of blisters each having a breachable lid and/or base that contains a dose of medicament for inhalation by a user of the device.

Oral or nasal delivery of a medicament using an inhalation device is a particularly attractive method of drug administration as these devices are relatively easy for a patient to use discreetly and in public. As well as delivering medicament to treat local diseases of the airway and other respiratory problems, they have more recently also been used to deliver drugs to the bloodstream via the lungs thereby avoiding the need for hypodermic injections.

It is common for dry powder formulations to be pre-packaged in individual doses, usually in the form of capsules or blisters which each contain a single dose of the powder which has been accurately and consistently measured. A blister is generally cold formed from a ductile foil laminate or a plastics material and includes a puncturable or peelable lid which is heat-sealed around the periphery of the blister during manufacture and after introduction of the dose into the blister. A foil blister is preferred over a polymer blister or gelatine capsule as each dose is protected from the ingress of water and penetration of gases such as oxygen in addition to being shielded from light and UV radiation all of which can have a detrimental effect on the delivery characteristics of the inhaler if a dose becomes exposed to them. Therefore, a blister offers excellent environmental protection to each individual drug dose.

Inhalation devices that receive a blister pack comprising a number of blisters each of which contain a pre-metered and individually packaged dose of the drug to be delivered are known. Actuation of the device causes a mechanism to breach or rupture a blister, such as by puncturing it or peeling the lid off, so that when the patient inhales, air is drawn through the blister entraining the dose therein that is then carried out of the blister through the device and via the patient's airway down into the lungs. Pressurized air or gas or other propellants may also be used to carry the dose out of the blister. Alternatively, the mechanism that punctures or opens the blister may also push or eject the dose out of the blister into a receptacle from which the dose may subsequently be inhaled.

It is advantageous for the inhaler to be capable of holding a number of doses to enable it to be used repeatedly over a period of time without the requirement to open and/or insert a blister into the device each time it is used. Therefore, many conventional devices include means for storing a number or strip of blisters each containing an individual dose of medicament. When a dose is to be inhaled, an indexing mechanism moves a previously emptied blister away from the opening mechanism so that a fresh one is moved into a position ready to be opened for inhalation of its contents.

An inhaler of the type described above is known from the Applicant's own co-pending international application no. PCT/GB2004/004416 filed on 18th October 2004 and claiming priority from GB application no. 0324358.1 filed 17th October 2003. This international application has been published as WO 2005/037353 A1.

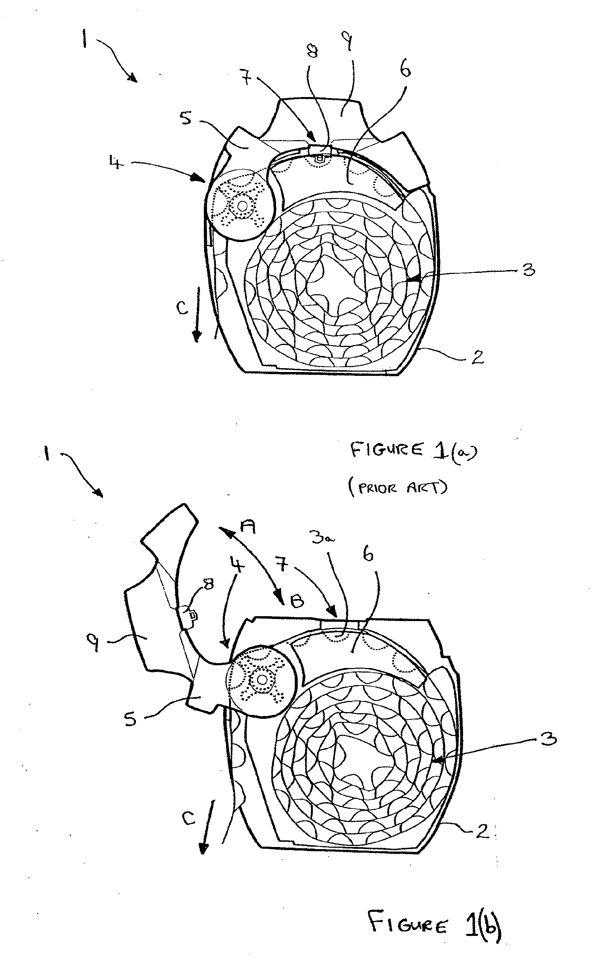

According to one embodiment described and claimed in WO 2005/037353 A1, and illustrated in Figures 1a and 1b of the accompanying drawings, an inhaler 1 has a housing 2 containing a coiled strip of blisters 3. An indexing mechanism 4 comprising a single actuating lever 5 that unwinds the coil 3 one blister at a time so that each blister passes over a blister locating chassis 6 and successively through a blister piercing station 7, when the actuator 5 is pivoted in a direction indicated by arrow "A" in Figure 1b. The blister 3a located at the blister piercing station 7 on each movement of the actuator 5 is pierced on the return stroke of the actuator 5 (in the direction indicated by arrow "B" in Figure 1b) by piercing elements 8 on the actuator 5 itself so that, when a user inhales through a mouthpiece 9, an airflow is generated within the blister 3a to entrain the dose contained therein and carry it out of the blister 3a via the mouthpiece 9 and into the user's airway.

Although the inhalation device 1 referred to above and described in the aforementioned publication has addressed many of the known problems associated with these types of devices, it is designed so as to store only a small number of used blisters 3 within the device so that, when that number of blisters 3 is exceeded, they extend out of the housing of the device 1 so that the user must separate those used blisters 3 from those unused blisters 3 that remain within the device 1 and discard the detached portion.

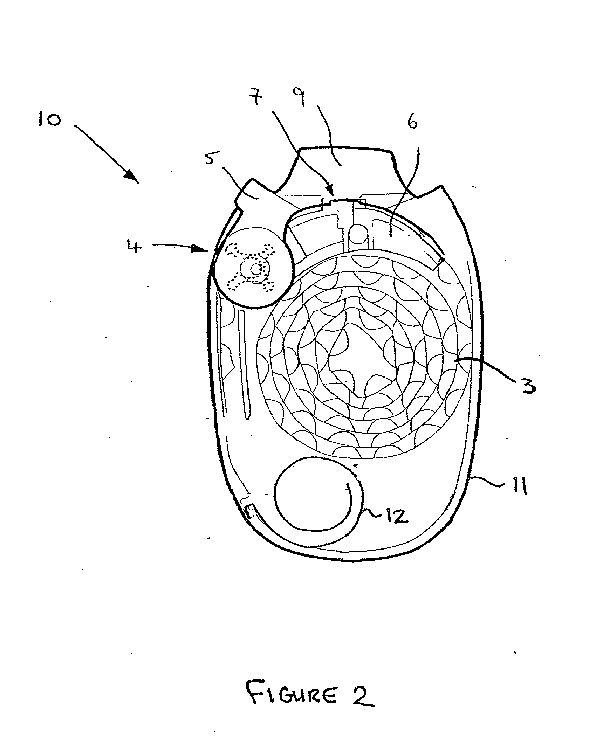

Although devices that eject used blisters have the advantage of being particularly small and lightweight, a fully integrated device has also been proposed. One such device is described in WO 2005/037353 A1 with reference to Figure 30. Another version has also been described in the Applicant's own earlier filed European Patent Application No. 07111998.6, filed on 6th July 2007, in which all the used blisters 3 are retained within the device 10 so that separation of used blisters 3 from those that remain in the device is no longer necessary. In a fully integrated device 10 of this type, one version of which is illustrated in Figure 2, the user no longer has to concern themselves with periodic detachment and disposal of a used portion of the blister strip 3 as all the used blisters 3 are retained within the housing 11 of the device 10. In the illustrated version of the device 10, the used blisters 3 are fed into a flexible spiral wound element 12 which coils the used portion of the strip 3 neatly within the housing 11 of the device 10. It will also be appreciated that any potential contamination of the fingers by residual drug remaining on the used blisters 3 can be avoided because there is no need for the user to come into contact with any of the used blisters 3.

A potential complication with a fully integrated device is that a small amount of the powdered dose, typically around 1% - 5%, may remain in each blister after inhalation. Furthermore, if a patient indexes the strip without having previously inhaled the dose in a blister that has been pierced or breached, the amount of residual powder will be substantial. This powder may escape from the opened blister and enter the housing or mechanism of the inhaler. It is therefore important to prevent the unused blisters from becoming contaminated with loose powder that could have a detrimental effect on the operation of the device and also result in the patient exceeding an intended dose, as they may inhale some of the residual powder as well as the contents of a pierced blister.

Furthermore, if the residual powder has been exposed to the atmosphere for a period of time, it may have also degraded making it unsuitable for inhalation.

The Applicant's aforementioned earlier filed application seeks to address the problem of powder contamination by separating the used and unused blister strips from each other by a flexible and/or movable dividing wall. The dividing wall is configured so that it extends across said space between said sidewalls of the inhaler to prevent passage of powdered dose between unused and used blister compartments. It is also known from the Applicant's own earlier European patent application No. 07111996.0, also filed on 6th July 2007, to provide a fixed wall between the used and unused blister compartments of the device.

Although the provision of a flexible/movable or fixed dividing wall is sufficient to substantially eliminate the problem of powder contamination, the present invention provides an alternative solution to the same problem and seeks to prevent residual powder from contaminating unused blisters remaining in the device and from being inhaled by a user. The present invention is not limited to a device in which all used blisters are retained within the housing and is also applicable to the device described with reference to Figure 1, in which used blisters are ejected from the device, as it prevents contact between the patient's fingers and any residual drug when the patient detaches a used portion of the strip.

An objective of the present invention is to effectively re-seal the punctured blisters after they have been pierced and indexed forward from their inhalation position or position in which they are aligned with the blister puncturing or opening member. By re-sealing a blister, any residual drug remaining in that blister is trapped within it and cannot escape from that blister to contaminate other unused blisters, components of the device or, a patient's fingers used to handle a used portion of a strip.

According to the invention, there is provided an inhaler comprising a housing to receive a strip of sealed blisters each containing a dose of medicament, means to move a strip received within the housing such that each blister is sequentially aligned with a blister opening member for opening said aligned blister to facilitate inhalation of said dose and, means for re-sealing opened blisters previously aligned with the blister opening member by applying adhesive tape to the strip.

The means for re-sealing opened blisters is advantageously configured to re-seal opened blisters in response to operation of the means for moving the strip.

In one embodiment, the means for re-sealing opened blisters includes a shaft in the housing to receive a reel of adhesive tape such that it can be unwound from the reel and adhered to a surface of the strip to re-seal opened blisters.

The shaft is preferably configured to receive a reel of adhesive tape freely mounted for rotation about the shaft.

In one embodiment, the means for re-sealing opened blisters preferably comprises a guide element parallel to but spaced from the shaft around which adhesive tape is passed as it is unwound from a reel and before being adhered to the surface of a strip.

The guide element may be fixed so that the adhesive tape slides over it as it is unwound from a reel.

The shaft preferably has an axis that lies parallel to but spaced from the surface of a strip received in the housing, said shaft being movably mounted to the housing.

The shaft may be mounted such that, when adhesive tape is pulled from a reel received on the shaft, the axis of the shaft is drawn closer to said surface.

In one embodiment, the inhaler may include biasing means to bias the shaft in a direction towards the surface of a strip.

The shaft may extend from one end of an arm pivotally mounted to the housing at its opposite end.

In one embodiment their may be guide slots in the housing to slideably receive the ends of the shaft. The guide slots can be configured such that the ends of the shaft slide in the slots such that the shaft moves towards the surface of a strip when adhesive tape is pulled from a reel.

According to one aspect, there is a reel of adhesive tape received in the housing of the inhaler according to the invention.

According to another aspect, there is a coiled strip of blisters received within the housing.

Preferably, the axis about which the reel is mounted for rotatation to unwind the adhesive tape is substantially parallel to the axis about which the coiled strip rotates to uncoil the strip during operation of the means for moving the strip within the housing.

In one embodiment, a leading end of the adhesive tape is unwound from the reel and adhered to a surface of the strip.

The reel of adhesive tape preferably has one adhesive side, said reel being wound such that said adhesive side faces inward towards the axis of the shaft. Alternatively, said reel can be wound such that said adhesive side faces outward away from the axis of the shaft.

According to another aspect of the invention, there is provided a method of assembling an inhaler comprising a housing to receive a strip of sealed blisters each containing a dose of medicament, the method including the step of placing a coiled strip of blisters in the housing together with a reel of adhesive tape, a leading end of said adhesive tape being unwound from the reel and adhered to the surface of the strip.

It will be appreciated that the inhaler of the invention may be either a passive or active device. In a passive device, the dose is entrained in a flow of air caused when the user inhales through the mouthpiece. However, in an active device, the inhaler would include means for generating a pressurised flow of gas or air through the blister to entrain the dose and carry it out of the blister through the mouthpiece and into the user's airway. In one embodiment, the inhaler may be provided with a source of pressurised gas or air within the housing.

Embodiments of the invention will now be described, by way of example only, with reference to Figures 4 to 7 of the accompanying drawings, in which:-
FIGURE 1a and 1b are side sectional views of a conventional inhalation device to show how the blisters of a strip are sequentially moved into alignment with a blister piercing station by movement of an actuator from the position shown in Figure 1a to the position shown in Figure 1b which drives an indexing wheel. A piercing head on the actuator pierces the lid of an aligned blister when the actuator is returned to its normal position as shown in Figure 1a;
FIGURE 2 is a side sectional view of a conventional fully integrated device in which all the used blisters are retained within the housing;
FIGURES 3a and 3b show a perspective view of a conventional coiled blister strip and a perspective view of a simplified view of a inhaler showing such a coiled blister strip located therein, respectively;
FIGURE 4 shows a perspective view of a coiled blister strip showing a reel of adhesive tape adjacent thereto and partially unwound so as to extend over a used portion of the blister strip;
FIGURE 5 shows the coiled blister strip and reel of adhesive tape received within the housing of an inhalation device, such as the device shown in Figure 3b;
FIGURE 6 shows a side sectional view of an inhalation device, similar to that shown in Figure 2, but incorporating the reel of adhesive tape; and
FIGURE 7 shows a perspective view of a coiled blister strip showing a reel of adhesive tape adjacent thereto according to another embodiment.

Reference is made throughout this specification to both "unused" and "used" blisters. It will be appreciated that "unused" blisters refer to those blisters that have not passed the blister piercing station and which remain intact with the dose contained therein. "Used" blisters refer to those blisters which have passed the blister piercing station in response to movement of the actuator by a user and which have been pierced to enable access to the dose contained therein to be obtained. Although in general, a "used" blister refers to a blister from which a dose has been inhaled, it should also be taken to include blisters which have passed the blister piercing station and have been pierced but which still contain either some or all of the dose contained therein. This may happen, for example, when a user moves the actuator to move the blister strip without inhaling the dose from a previously pierced blister.

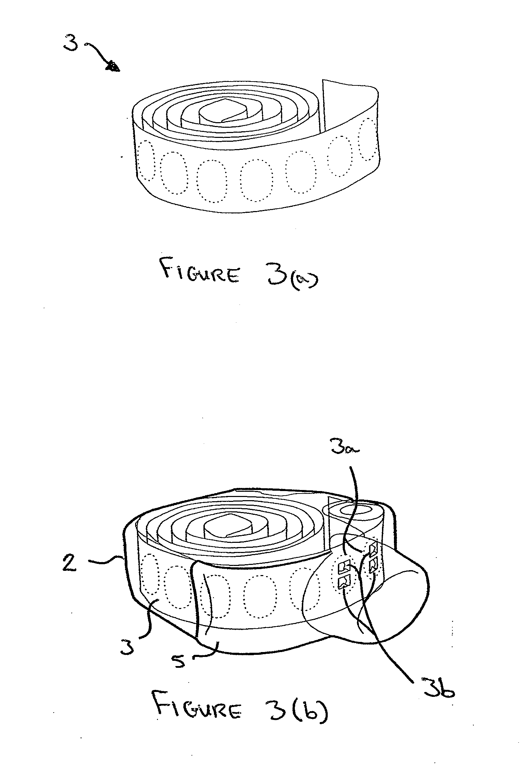

Referring now to Figure 3a and 3b, there is shown a conventional coiled strip of blisters and, the coiled strip of blisters located within a housing of an inhalation device of the type already described with reference to Figures 1a and 1b, respectively. The components of the device are shown as being transparent to enable the strip to be clearly seen. The indexing mechanism and other parts of the device are omitted in the interest of clarity. As can be seen in Figure 3b, two of the blisters 3a have passed the blister piercing station and two openings 3b have been pierced in each of them. It is through these openings that residual powder can escape and contaminate the unused portion of the strip 3 and/or coat the interior surface and components of the device.

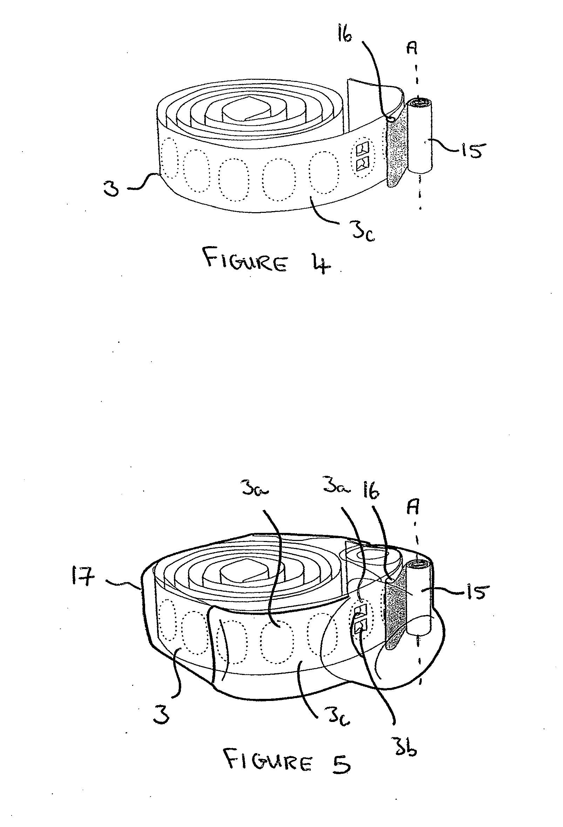

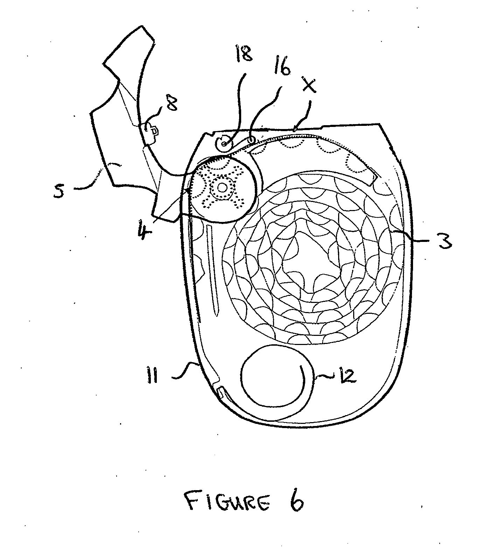

Figure 4 illustrates the coiled strip of blisters 3 of Figure 3a but which has been modified by the addition of a reel 15 of adhesive tape which is located adjacent to the strip 3 with its axis "A" parallel to an upper surface 3c of the strip 3. One end of the adhesive tape extending from the reel has been fed around a guide post or shaft 16 from where it passes onto the upper surface 3c of the strip so as to adhere to and cover the upper surface and seal the opened blisters 3. It is envisaged that the reel 15 and strip 3 may be attached to each other and inserted into the housing of the inhalation device together. In this instance, a portion of the end of the tape 15 may be adhered to a portion of the leading end of the blister strip 3 prior to insertion, as shown in Figure 4. This ensures that the tape 15 and strip 3 are aligned correctly. When the combined blister strip 3 and tape reel 15 are inserted into the housing 17 of a device, as shown in Figure 5, the reel 15 may be slid over an axle 18 (see Figure 6) and the tape 15 around the guide post 16, both of which may form part of the housing itself. The axle 18 is such that the reel 15 can freely rotate around it so that tape is dispensed from the reel 15 when the strip 3 is indexed so as to cover the upper surface 3c of the strip 3 and, more importantly, the opened blisters. The adhesive tape 15 is effectively dragged or pulled from the reel causing the reel 15 to rotate about its axis A, unwinding the tape 15and sticking it to the upper surface of the strip 3, thereby sealing the pierced blisters.

Although it is preferable for the tape to be pulled from the reel 15 during indexing of the strip 3, it is also envisaged that a separate drive element (not shown) could be provided to unwind the reel 15.

The guide post 16 is preferably fixed so that the tape slides over it, although it may also rotate. The post 16 may also be mounted for movement in a direction at right-angles to the axis of the post 16 towards and away from the surface of the strip 3, so as to press the tape 15 into contact with the strip 3. In one embodiment, the post 16 may be biased in a direction towards the surface of the strip 3.

As the tape passes over the guide post 16, the angle at which the tape 15 is applied to the strip 3 remains constant despite changes in the diameter of the tape remaining on the reel 15.

A fully integrated device, similar to that shown in Figure 2, is illustrated in Figure 6. However, it will be appreciated that the device has been modified according to the invention by the provision of a reel of adhesive tape, as described with reference to Figures 4 and 5. It will be appreciated that the reel 15 is placed as close as possible to the location marked "X" at which the blisters are pierced so that the tape 15 is dispensed to cover pierced blisters as soon as they move away from the piercing position to thereby seal the opened blisters and minimise or prevent the escape of residual powder.

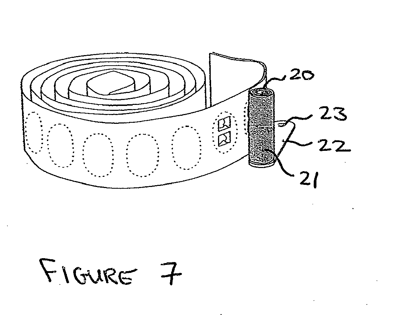

An alternative embodiment of the invention is illustrated in Figure 7. In this embodiment, an axle 20 for the reel 21 is mounted to and extends from the free end of a tensioning arm 22 which is pivotally mounted to a fixed pivot 23 on the housing at its opposite end. The arm pivot is so positioned so that the reel 21 is biased into contact with the surface of the blister strip 3c by the motion of the blister strip 3c, as it is indexed, pulling on the end of the reel of tape, 21. In addition an active biasing element such as a spring could be incorporated to further bias the arm 22 towards the blister strip surface 3c. It will be appreciated that, in this embodiment the tape is reverse wound on the reel with the sticky side facing out, as opposed to the more conventionally wound reel in the embodiment of figure 5. The tape is applied directly from the reel 21 to the surface 3c of the blister strip 3, rather than passing over a guide post. As the strip 3 is indexed, the adhesive tape is dragged from the reel 21 to cause the tensioning arm 22 to rotate about its fixed pivot 23 to thereby draw the reel 21 further against the surface 3c of the strip 3 to impart additional force normal to the surface of the strip 3 and thereby assist adhesion of the adhesive tape to the surface of the strip 3. The same effect may be obtained in another, unillustrated embodiment, by mounting the axle of the reel in blind slots in the housing which are configured to allow the reel to be drawn towards the blister strip as it is indexed forward.

Although Figure 7 shows a fully integrated device with the tape dispensing arrangement of Figures 4 and 5, it will be appreciated that the tape dispensing arrangement described in the previous paragraph may also be employed in such a device shown in Figure 7.

It will be appreciated that, in the fully integrated device employing a spiral wound element 12 to receive the used portion of the strip 3, the upper, non-adhesive, surface of the tape may have low friction characteristics or be especially smooth so as to enhance the movement of the used portion of the strip against the spiral wound element and thereby minimise friction.

A variety of medicaments may be administered alone by using inhalers of the invention. Specific active agents or drugs that may be used include, but are not limited to, agents of one or more of the following classes listed below.
1) Adrenergic agonists such as, for example, amphetamine, apraclonidine, bitolterol, clonidine, colterol, dobutamine, dopamine, ephedrine, epinephrine, ethylnorepinephrine, fenoterol, formoterol, guanabenz, guanfacine, hydroxyamphetamine, isoetharine, isoproterenol, isotharine, mephenterine, metaraminol, methamphetamine, methoxamine, methpentermine, methyldopa, methylphenidate, metaproterenol, metaraminol, mitodrine, naphazoline, norepinephrine, oxymetazoline, pemoline, phenylephrine, phenylethylamine, phenylpropanolamine, pirbuterol, prenalterol, procaterol, propylhexedrine, pseudoephedrine, ritodrine, salbutamol, salmeterol, terbutaline, tetrahydrozoline, tramazoline, tyramine and xylometazoline.
2) Adrenergic antagonists such as, for example, acebutolol, alfuzosin, atenolol, betaxolol, bisoprolol, bopindolol, bucindolol, bunazosin, butyrophenones, carteolol, carvedilol, celiprolol, chlorpromazine, doxazosin, ergot alkaloids, esmolol, haloperidol, indoramin, ketanserin, labetalol, levobunolol, medroxalol, metipranolol, metoprolol, nebivolol, nadolol, naftopidil, oxprenolol, penbutolol, phenothiazines, phenoxybenzamine, phentolamine, pindolol, prazosin, propafenone, propranolol, sotalol, tamsulosin, terazosin, timolol, tolazoline, trimazosin, urapidil and yohimbine.
3) Adrenergic neurone blockers such as, for example, bethanidine, debrisoquine, guabenxan, guanadrel, guanazodine, guanethidine, guanoclor and guanoxan.
4) Drugs for treatment of addiction, such as, for example, buprenorphine.
5) Drugs for treatment of alcoholism, such as, for example, disulfiram, naloxone and naltrexone.
6) Drugs for Alzheimer's disease management, including acetylcholinesterase inhibitors such as, for example, donepezil, galantamine, rivastigmine and tacrin.
7) Anaesthetics such as, for example amethocaine, benzocaine, bupivacaine, hydrocortisone, ketamine, lignocaine, methylprednisolone, prilocaine, proxymetacaine, ropivacaine and tyrothricin.
8) Angiotensin converting enzyme inhibitors such as, for example, captopril, cilazapril, enalapril, fosinopril, imidapril hydrochloride, lisinopril, moexipril hydrochloride, perindopril, quinapril, ramipril and trandolapril.
9) Angiotensin II receptor blockers, such as, for example, candesartan, cilexetil, eprosartan, irbesartan, losartan, medoxomil, olmesartan, telmisartan and valsartan.
10) Antiarrhythmics such as, for example, adenosine, amidodarone, disopyramide, flecainide acetate, lidocaine hydrochloride, mexiletine, procainamide, propafenone and quinidine.
11) Antibiotic and antibacterial agents (including the beta-lactams, fluoroquinolones, ketolides, macrolides, sulphonamides and tetracyclines) such as, for example, aclarubicin, amoxicillin, amphotericin, azithromycin, aztreonam chlorhexidine, clarithromycin, clindamycin, colistimethate, dactinomycin, dirithromycin, doripenem, erythromycin, fusafungine, gentamycin, metronidazole, mupirocin, natamycin, neomycin, nystatin, oleandomycin, pentamidine, pimaricin, probenecid, roxithromycin, sulphadiazine and triclosan.
12) Anti-clotting agents such as, for example, abciximab, acenocoumarol, alteplase, aspirin, bemiparin, bivalirudin, certoparin, clopidogrel, dalteparin, danaparoid, dipyridamole, enoxaparin, epoprostenol, eptifibatide, fondaparin, heparin (including low molecular weight heparin), heparin calcium, lepirudin, phenindione, reteplase, streptokinase, tenecteplase, tinzaparin, tirofiban and warfarin.
13) Anticonvulsants such as, for example, GABA analogs including tiagabine and vigabatrin; barbiturates including pentobarbital; benzodiazepines including alprazolam, chlordiazepoxide, clobazam, clonazepam, diazepam, flurazepam, lorazepam, midazolam, oxazepam and zolazepam; hydantoins including phenytoin; phenyltriazines including lamotrigine; and miscellaneous anticonvulsants including acetazolamide, carbamazepine, ethosuximide, fosphenytoin, gabapentin, levetiracetam, oxcarbazepine, piracetam, pregabalin, primidone, sodium valproate, topiramate, valproic acid and zonisamide.
14) Antidepressants such as, for example, tricyclic and tetracyclic antidepressants including amineptine, amitriptyline (tricyclic and tetracyclic amitryptiline), amoxapine, butriptyline, cianopramine, clomipramine, demexiptiline, desipramine, dibenzepin, dimetacrine, dosulepin, dothiepin, doxepin, imipramine, iprindole, levoprotiline, lofepramine, maprotiline, melitracen, metapramine, mianserin, mirtazapine, nortryptiline, opipramol, propizepine, protriptyline, quinupramine, setiptiline, tianeptine and trimipramine; selective serotonin and noradrenaline reuptake inhibitors (SNRIs) including clovoxamine, duloxetine, milnacipran and venlafaxine; selective serotonin reuptake inhibitors (SSRIs) including citalopram, escitalopram, femoxetine, fluoxetine, fluvoxamine, ifoxetine, milnacipran, nomifensine, oxaprotiline, paroxetine, sertraline, sibutramine, venlafaxine, viqualine and zimeldine; selective noradrenaline reuptake inhibitors (NARIs) including demexiptiline, desipramine, oxaprotiline and reboxetine; noradrenaline and selective serotonin reuptake inhibitors (NASSAs) including mirtazapine; monoamine oxidase inhibitors (MAOIs) including amiflamine, brofaromine, clorgyline, α-ethyltryptamine, etoperidone, iproclozide, iproniazid, isocarboxazid, mebanazine, medifoxamine, moclobemide, nialamide, pargyline, phenelzine, pheniprazine, pirlindole, procarbazine, rasagiline, safrazine, selegiline, toloxatone and tranylcypromine; muscarinic antagonists including benactyzine and dibenzepin; azaspirones including buspirone, gepirone, ipsapirone, tandospirone and tiaspirone; and other antidepressants including acetaphenazine, ademetionine, S-adenosylmethionine, adrafinil, amesergide, amineptine, amperozide, benactyzine, benmoxine, binedaline, bupropion, carbamazepine, caroxazone, cericlamine, cotinine, fezolamine, flupentixol, idazoxan, kitanserin, levoprotiline, lithium salts, maprotiline, medifoxamine, methylphenidate, metralindole, minaprine, nefazodone, nisoxetine, nomifensine, oxaflozane, oxitriptan, phenyhydrazine, rolipram, roxindole, sibutramine, teniloxazine, tianeptine, tofenacin, trazadone, tryptophan, viloxazine and zalospirone.
15) Anticholinergic agents such as, for example, atropine, benzatropine, biperiden, cyclopentolate, glycopyrrolate, hyoscine, ipratropium bromide, orphenadine hydrochloride, oxitroprium bromide, oxybutinin, pirenzepine, procyclidine, propantheline, propiverine, telenzepine, tiotropium, trihexyphenidyl, tropicamide and trospium.
16) Antidiabetic agents such as, for example, pioglitazone, rosiglitazone and troglitazone.
17) Antidotes such as, for example, deferoxamine, edrophonium chloride, fiumazenil, nalmefene, naloxone, and naltrexone.
18) Anti-emetics such as, for example, alizapride, azasetron, benzquinamide, bestahistine, bromopride, buclizine, chlorpromazine, cinnarizine, clebopride, cyclizine, dimenhydrinate, diphenhydramine, diphenidol, domperidone, dolasetron, dronabinol, droperidol, granisetron, hyoscine, lorazepam, metoclopramide, metopimazine, nabilone, ondansetron, palonosetron, perphenazine, prochlorperazine, promethazine, scopolamine, triethylperazine, trifluoperazine, triflupromazine, trimethobenzamide and tropisetron.
19) Antihistamines such as, for example, acrivastine, astemizole, azatadine, azelastine, brompheniramine, carbinoxamine, cetirizine, chlorpheniramine, cinnarizine, clemastine, cyclizine, cyproheptadine, desloratadine, dexmedetomidine, diphenhydramine, doxylamine, fexofenadine, hydroxyzine, ketotifen, levocabastine, loratadine, mizolastine, promethazine, pyrilamine, terfenadine and trimeprazine.
20) Anti-infective agents such as, for example, antivirals (including nucleoside and non-nucleoside reverse transcriptase inhibitors and protease inhibitors) including aciclovir, adefovir, amantadine, cidofovir, efavirenz, famiciclovir, foscarnet, ganciclovir, idoxuridine, indinavir, inosine pranobex, lamivudine, nelfinavir, nevirapine, oseltamivir, palivizumab, penciclovir, pleconaril, ribavirin, rimantadine, ritonavir, ruprintrivir, saquinavir, stavudine, valaciclovir, zalcitabine, zanamivir, zidovudine and interferons; AIDS adjunct agents including dapsone; aminoglycosides including tobramycin; antifungals including amphotericin, caspofungin, clotrimazole, econazole nitrate, fluconazole, itraconazole, ketoconazole, miconazole, nystatin, terbinafine and voriconazole; anti-malarial agents including quinine; antituberculosis agents including capreomycin, ciprofloxacin, ethambutol, meropenem, piperacillin, rifampicin and vancomycin; beta-lactams including cefazolin, cefmetazole, cefoperazone, cefoxitin, cephacetrile, cephalexin, cephaloglycin and cephaloridine; cephalosporins, including cephalosporin C and cephalothin; cephamycins such as cephamycin A, cephamycin B, cephamycin C, cephapirin and cephradine; leprostatics such as clofazimine; penicillins including amoxicillin, ampicillin, amylpenicillin, azidocillin, benzylpenicillin, carbenicillin, carfecillin, carindacillin, clometocillin, cloxacillin, cyclacillin, dicloxacillin, diphenicillin, heptylpenicillin, hetacillin, metampicillin, methicillin, nafcillin, 2-pentenylpenicillin, penicillin N, penicillin O, penicillin S and penicillin V; quinolones including ciprofloxacin, clinafloxacin, difloxacin, grepafloxacin, norfloxacin, ofloxacine and temafloxacin; tetracyclines including doxycycline and oxytetracycline; miscellaneous anti-infectives including linezolide, trimethoprim and sulfamethoxazole.
21) Anti-neoplastic agents such as, for example, droloxifene, tamoxifen and toremifene.
22) Antiparkisonian drugs such as, for example, amantadine, andropinirole, apomorphine, baclofen, benserazide, biperiden, benztropine, bromocriptine, budipine, cabergoline, carbidopa, eliprodil, entacapone, eptastigmine, ergoline, galanthamine, lazabemide, levodopa, lisuride, mazindol, memantine, mofegiline, orphenadrine, trihexyphenidyl, pergolide, piribedil, pramipexole, procyclidine, propentofylline, rasagiline, remacemide, ropinerole, selegiline, spheramine, terguride and tolcapone.
23) Antipsychotics such as, for example, acetophenazine, alizapride, amisulpride, amoxapine, amperozide, aripiprazole, benperidol, benzquinamide, bromperidol, buramate, butaclamol, butaperazine, carphenazine, carpipramine, chlorpromazine, chlorprothixene, clocapramine, clomacran, clopenthixol, clospirazine, clothiapine, clozapine, cyamemazine, droperidol, flupenthixol, fluphenazine, fluspirilene, haloperidol, loxapine, melperone, mesoridazine, metofenazate, molindrone, olanzapine, penfluridol, pericyazine, perphenazine, pimozide, pipamerone, piperacetazine, pipotiazine, prochlorperazine, promazine, quetiapine, remoxipride, risperidone, sertindole, spiperone, sulpiride, thioridazine, thiothixene, trifluperidol, triflupromazine, trifluoperazine, ziprasidone, zotepine and zuclopenthixol; phenothiazines including aliphatic compounds, piperidines and piperazines; thioxanthenes, butyrophenones and substituted benzamides.
24) Antirheumatic agents such as, for example, diclofenac, heparinoid, hydroxychloroquine and methotrexate, leflunomide and teriflunomide.
25) Anxiolytics such as, for example, adinazolam, alpidem, alprazolam, alseroxlon, amphenidone, azacyclonol, bromazepam, bromisovalum, buspirone, captodiamine, capuride, carbcloral, carbromal, chloral betaine, chlordiazepoxide, clobenzepam, enciprazine, flesinoxan, flurazepam, hydroxyzine, ipsapiraone, lesopitron, loprazolam, lorazepam, loxapine, mecloqualone, medetomidine, methaqualone, methprylon, metomidate, midazolam, oxazepam, propanolol, tandospirone, trazadone, zolpidem and zopiclone.
26) Appetite stimulants such as, for example, dronabinol.
27) Appetite suppressants such as, for example, fenfluramine, phentermine and sibutramine; and anti-obesity treatments such as, for example, pancreatic lipase inhibitors, serotonin and norepinephrine re-uptake inhibitors, and anti-anorectic agents.
28) Benzodiazepines such as, for example, alprazolam, bromazepam, brotizolam, chlordiazepoxide, clobazam, clonazepam, clorazepate, demoxepam, diazepam, estazolam, flunitrazepam, flurazepam, halazepam, ketazolam, loprazolam, lorazepam, lormetazepam, medazepam, midazolam, nitrazepam, nordazepam, oxazepam, prazepam, quazepam, temazepam and triazolam.
29) Bisphosphonates such as, for example, alendronate sodium, sodium clodronate, etidronate disodium, ibandronic acid, pamidronate disodium, isedronate sodium, tiludronic acid and zoledronic acid.
30) Blood modifiers such as, for example, cilostazol and dipyridamol, and blood factors.
31) Cardiovascular agents such as, for example, acebutalol, adenosine, amiloride, amiodarone, atenolol, benazepril, bisoprolol, bumetanide, candesartan, captopril, clonidine, diltiazem, disopyramide, dofetilide, doxazosin, enalapril, esmolol, ethacrynic acid, flecanide, furosemide, gemfibrozil, ibutilide, irbesartan, labetolol, losartan, lovastatin, metolazone, metoprolol, mexiletine, nadolol, nifedipine, pindolol, prazosin, procainamide, propafenone, propranolol, quinapril, quinidine, ramipril, sotalol, spironolactone, telmisartan, tocainide, torsemide, triamterene, valsartan and verapamil.
32) Calcium channel blockers such as, for example, amlodipine, bepridil, diltiazem, felodipine, flunarizine, gallopamil, isradipine, lacidipine, lercanidipine, nicardipine, nifedipine, nimodipine and verapamil.
33) Central nervous system stimulants such as, for example, amphetamine, brucine, caffeine, dexfenfluramine, dextroamphetamine, ephedrine, fenfluramine, mazindol, methyphenidate, modafmil, pemoline, phentermine and sibutramine.
34) Cholesterol-lowering drugs such as, for example, acipimox, atorvastatin, ciprofibrate, colestipol, colestyramine, bezafibrate, ezetimibe, fenofibrate, fluvastatin, gemfibrozil, ispaghula, nictotinic acid, omega-3 triglycerides, pravastatin, rosuvastatin and simvastatin.
35) Drugs for cystic fibrosis management such as, for example, Pseudomonas aeruginosa infection vaccines (eg Aerugen™, alpha 1-antitripsin, amikacin, cefadroxil, denufosol, duramycin, glutathione, mannitol, and tobramycin.
36) Diagnostic agents such as, for example, adenosine and aminohippuric acid.
37) Dietary supplements such as, for example, melatonin and vitamins including vitamin E.
38) Diuretics such as, for example, amiloride, bendroflumethiazide, bumetanide, chlortalidone, cyclopenthiazide, furosemide, indapamide, metolazone, spironolactone and torasemide.
39) Dopamine agonists such as, for example, amantadine, apomorphine, bromocriptine, cabergoline, lisuride, pergolide, pramipexole and ropinerole.
40) Drugs for treating erectile dysfunction, such as, for example, apomorphine, apomorphine diacetate, moxisylyte, phentolamine, phosphodiesterase type 5 inhibitors, such as sildenafil, tadalafil, vardenafil and yohimbine.
41) Gastrointestinal agents such as, for example, atropine, hyoscyamine, famotidine, lansoprazole, loperamide, omeprazole and rebeprazole.
42) Hormones and analogues such as, for example, cortisone, epinephrine, estradiol, insulin, Ostabolin-C, parathyroid hormone and testosterone.
43) Hormonal drugs such as, for example, desmopressin, lanreotide, leuprolide, octreotide, pegvisomant, protirelin, salcotonin, somatropin, tetracosactide, thyroxine and vasopressin.
44) Hypoglycaemics such as, for example, sulphonylureas including glibenclamide, gliclazide, glimepiride, glipizide and gliquidone; biguanides including metformin; thiazolidinediones including pioglitazone, rosiglitazone, nateglinide, repaglinide and acarbose.
45) Immunoglobulins.
46) Immunomodulators such as, for example, interferon (e.g. interferon beta-1a and interferon beta-1b) and glatiramer.
47) Immunosupressives such as, for example, azathioprine, cyclosporin, mycophenolic acid, rapamycin, sirolimus and tacrolimus.
48) Mast cell stabilizers such as, for example, cromoglycate, iodoxamide, nedocromil, ketotifen, tryptase inhibitors and pemirolast.
49) Drugs for treatment of migraine headaches such as, for example, almotriptan, alperopride, amitriptyline, amoxapine, atenolol, clonidine, codeine, coproxamol, cyproheptadine, dextropropoxypene, dihydroergotamine, diltiazem, doxepin, ergotamine, eletriptan, fluoxetine, frovatriptan, isometheptene, lidocaine, lisinopril, lisuride, loxapine, methysergide, metoclopramide, metoprolol, nadolol, naratriptan, nortriptyline, oxycodone, paroxetine, pizotifen, pizotyline, prochlorperazine propanolol, propoxyphene, protriptyline, rizatriptan, sertraline, sumatriptan, timolol, tolfenamic acid, tramadol, verapamil, zolmitriptan, and non-steroidal anti-inflammatory drugs.
50) Drugs for treatment of motion sickness such as, for example, diphenhydramine, promethazine and scopolamine.
51) Mucolytic agents such as N-acetylcysteine, ambroxol, amiloride, dextrans, heparin, desulphated heparin, low molecular weight heparin and recombinant human DNase.
52) Drugs for multiple sclerosis management such as, for example, bencyclane, methylprednisolone, mitoxantzone and prednisolone.
53) Muscle relaxants such as, for example, baclofen, chlorzoxazone, cyclobenzaprine, methocarbamol, orphenadrine, quinine and tizanidine.
54) NMDA receptor antagonists such as, for example, mementine.
55) Nonsteroidal anti-inflammatory agents such as, for example, aceclofenac, acetaminophen, alminoprofen, amfenac, aminopropylon, amixetrine, aspirin, benoxaprofen, bromfenac, bufexamac, carprofen, celecoxib, choline, cinchophen, cinmetacin, clometacin, clopriac, diclofenac, diclofenac sodium, diflunisal, ethenzamide, etodolac, etoricoxib, fenoprofen, flurbiprofen, ibuprofen, indomethacin, indoprofen, ketoprofen, ketorolac, loxoprofen, mazipredone, meclofenamate, mefenamic acid, meloxicam, nabumetone, naproxen, nimesulide, parecoxib, phenylbutazone, piroxicam, pirprofen, rofecoxib, salicylate, sulindac, tiaprofenic acid, tolfenamate, tolmetin and valdecoxib.
56) Nucleic-acid medicines such as, for example, oligonucleotides, decoy nucleotides, antisense nucleotides and other gene-based medicine molecules.
57) Opiates and opioids such as, for example, alfentanil, allylprodine, alphaprodine, anileridine, benzylmorphine, bezitramide, buprenorphine, butorphanol, carbiphene, cipramadol, clonitazene, codeine, codeine phosphate, dextromoramide, dextropropoxyphene, diamorphine, dihydrocodeine, dihydromorphine, diphenoxylate, dipipanone, fentanyl, hydromorphone, L-alpha acetyl methadol, levorphanol, lofentanil, loperamide, meperidine, meptazinol, methadone, metopon, morphine, nalbuphine, nalorphine, oxycodone, papaveretum, pentazocine, pethidine, phenazocine, pholcodeine, remifentanil, sufentanil, tramadol, and combinations thereof with an antiemetic.
58) Opthalmic preparations such as, for example, betaxolol and ketotifen.
59) Osteoporosis preparations such as, for example, alendronate, estradiol, estropitate, raloxifene and risedronate.
60) Other analgesics such as, for example, apazone, benzpiperylon, benzydamine, caffeine, cannabinoids, clonixin, ethoheptazine, flupirtine, nefopam, orphenadrine, pentazocine, propacetamol and propoxyphene.
61) Other anti-inflammatory agents such as, for example, B-cell inhibitors, p38 MAP kinase inhibitors and TNF inhibitors.
62) Phosphodiesterase inhibitors such as, for example, non-specific phosphodiesterase inhibitors including theophylline, theobromine, IBMX, pentoxifylline and papaverine; phosphodiesterase type 3 inhibitors including bipyridines such as milrinone, amrinone and olprinone; imidazolones such as piroximone and enoximone; imidazolines such as imazodan and 5-methyl-imazodan; imidazo-quinoxalines; and dihydropyridazinones such as indolidan and LY181512 (5-(6-oxo-1,4,5,6-tetrahydro-pyridazin-3-yl)-1,3-dihydro-indol-2-one); dihydroquinolinone compounds such as cilostamide, cilostazol, and vesnarinone; motapizone; phosphodiesterase type 4 inhibitors such as cilomilast, etazolate, rolipram, oglemilast, roflumilast, ONO 6126, tolafentrine and zardaverine, and including quinazolinediones such as nitraquazone and nitraquazone analogs; xanthine derivatives such as denbufylline and arofylline; tetrahydropyrimidones such as atizoram; and oxime carbamates such as filaminast; and phosphodiesterase type 5 inhibitors including sildenafil, zaprinast, vardenafil, tadalafil, dipyridamole, and the compounds described in WO 01/19802, particularly (S)-2-(2-hydroxymethyl-1-pyrrolidinyl)-4-(3-chloro-4-memoxy-benzylamino)-5-[N-(2-pyrimidinylmethyl)carbamoyl]pyrimidine, 2-(5,6,7,8-tetrahydro-1, 7-naphthyridin-7-y1)-4-(3-chloro-4-methoxybenzylamino)-5-[N-(2-morpholinoethyl)carbamoyl]-pyrimidine, and (S)-2-(2-hydroxymethyl-1-pyrrolidinyl)-4-(3-chloro-4-methoxy-benzylamino)-5-[N-(1,3,5-trimethyl-4-pyrazolyl)carbamoyl]-pyrimidine).
63) Potassium channel modulators such as, for example, cromakalim, diazoxide, glibenclamide, levcromakalim, minoxidil, nicorandil and pinacidil.
64) Prostaglandins such as, for example, alprostadil, dinoprostone, epoprostanol and misoprostol.
65) Respiratory agents and agents for the treatment of respiratory diseases including bronchodilators such as, for example, the β2-agonists bambuterol, bitolterol, broxaterol, carmoterol, clenbuterol, fenoterol, formoterol, indacaterol, levalbuterol, metaproterenol, orciprenaline, picumeterol, pirbuterol, procaterol, reproterol, rimiterol, salbutamol, salmeterol, terbutaline and the like; inducible nitric oxide synthase (iNOS) inhibitors; the antimuscarinics ipratropium, ipratropium bromide, oxitropium, tiotropium, glycopyrrolate and the like; the xanthines aminophylline, theophylline and the like; adenosine receptor antagonists, cytokines such as, for example, interleukins and interferons; cytokine antagonists and chemokine antagonists including cytokine synthesis inhibitors, endothelin receptor antagonists, elastase inhibitors, integrin inhibitors, leukotrine receptor antagonists, prostacyclin analogues, and ablukast, ephedrine, epinephrine, fenleuton, iloprost, iralukast, isoetharine, isoproterenol, montelukast, ontazolast, pranlukast, pseudoephedrine, sibenadet, tepoxalin, verlukast, zafirlukast and zileuton.
66) Sedatives and hypnotics such as, for example, alprazolam, butalbital, chlordiazepoxide, diazepam, estazolam, flunitrazepam, flurazepam, lorazepam, midazolam, temazepam, triazolam, zaleplon, zolpidem, and zopiclone.
67) Serotonin agonists such as, for example, 1-(4-bromo-2,5-dimethoxyphenyl)-2-aminopropane, buspirone, m-chlorophenylpiperazine, cisapride, ergot alkaloids, gepirone, 8-hydroxy-(2-N,N-dipropylamino)-tetraline, ipsaperone, lysergic acid diethylamide, 2-methyl serotonin, mezacopride, sumatriptan, tiaspirone, trazodone and zacopride.
68) Serotonin antagonists such as, for example, amitryptiline, azatadine, chlorpromazine, clozapine, cyproheptadine, dexfenfluramine, R(+)-α-(2,3-dimethoxyphenyl)-1-[2-(4-fluorophenyl)ethyl]-4-piperidine-methanol, dolasetron, fenclonine, fenfluramine, granisetron, ketanserin, methysergide, metoclopramide, mianserin, ondansetron, risperidone, ritanserin, trimethobenzamide and tropisetron.
69) Steroid drugs such as, for example, alcometasone, beclomethasone, beclomethasone dipropionate, betamethasone, budesonide, butixocort, ciclesonide, clobetasol, deflazacort, diflucortolone, desoxymethasone, dexamethasone, fludrocortisone, flunisolide, fluocinolone, fluometholone, fluticasone, fluticasone proprionate, hydrocortisone, methylprednisolone, mometasone, nandrolone decanoate, neomycin sulphate, prednisolone, rimexolone, rofleponide, triamcinolone and triamcinolone acetonide.
70) Sympathomimetic drugs such as, for example, adrenaline, dexamfetamine, dipirefin, dobutamine, dopamine, dopexamine, isoprenaline, noradrenaline, phenylephrine, pseudoephedrine, tramazoline and xylometazoline.
71) Nitrates such as, for example, glyceryl trinitrate, isosorbide dinitrate and isosorbide mononitrate.
72) Skin and mucous membrane agents such as, for example, bergapten, isotretinoin and methoxsalen.
73) Smoking cessation aids such as, for example, bupropion, nicotine and varenicline.
74) Drugs for treatment of Tourette's syndrome such as, for example, pimozide.
75) Drugs for treatment of urinary tract infections such as, for example, darifenicin, oxybutynin, propantheline bromide and tolteridine.
76) Vaccines.
77) Drugs for treating vertigo such as, for example, betahistine and meclizine.
78) Therapeutic proteins and peptides such as acylated insulin, glucagon, glucagon-like peptides, exendins, insulin, insulin analogues, insulin aspart, insulin detemir, insulin glargine, insulin glulisine, insulin lispro, insulin zinc, isophane insulins, neutral, regular and insoluble insulins, and protamine zinc insulin.
79) Anticancer agents such as, for example, anthracyclines, doxorubicin, idarubicin, epirubicin, methotrexate, taxanes, paclitaxel, docetaxel, cisplatin, vinca alkaloids, vincristine and 5-fluorouracil.
80) Pharmaceutically acceptable salts or derivatives of any of the foregoing.

It should be noted that drugs listed above under a particular indication or class may also find utility in other indications. A plurality of active agents can be employed in the practice of the present invention. An inhaler according to the invention may also be used to deliver combinations of two or more different active agents or drugs. Specific combinations of two medicaments which may be mentioned include combinations of steroids and β₂-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and formoterol; ciclesonide and salmeterol; mometasone and formoterol; and mometasone and salmeterol. Specifically, inhalers according to the invention may also be used to deliver combinations of three different active agents or drugs.

It will be clear to a person of skill in the art that, where appropriate, the active agents or drugs may be linked to a carrier molecule or molecules and/or used in the form of prodrugs, salts, as esters, or as solvates to optimise the activity and/or stability of the active agent or drug.

Anticholinergic agents are referred to above (see No. 15). It is also envisaged that the pharmaceutical composition may comprise one or more, preferably one, anticholinergic 1, optionally in combination with a pharmaceutically acceptable excipient.

The anticholinergic 1 can be selected from the group consisting of
a) tiotropium salts 1a,
b) compounds of formula 1c 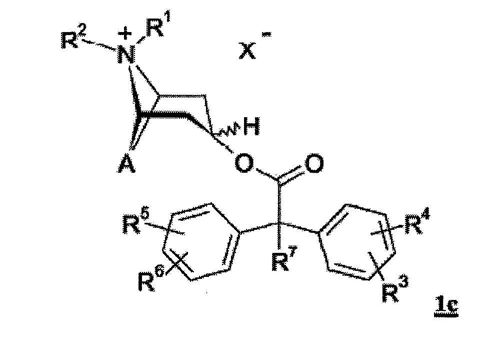 wherein
   A denotes a double-bonded group selected from among 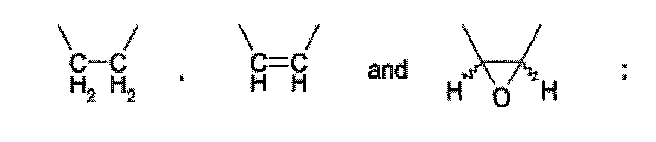 **X⁻** denotes an anion with a single negative charge, preferably an anion selected from the group consisting of fluoride, chloride, bromide, iodide, sulphate, phosphate, methanesulphonate, nitrate, maleate, acetate, citrate, fumarate, tartrate, oxalate, succinate, benzoate and p-toluenesulphonate,
   R¹ and R² which may be identical or different denote a group selected from among methyl, ethyl, n-propyl and iso-propyl, which may optionally be substituted by hydroxy or fluorine, preferably unsubstituted methyl;
   R³, R⁴, R⁵ and R⁶, which may be identical or different, denote hydrogen, methyl, ethyl, methyloxy, ethyloxy, hydroxy, fluorine, chlorine, bromine, CN, CF₃ or NO₂;
   R⁷ denotes hydrogen, methyl, ethyl, methyloxy, ethyloxy, -CH₂-F, -CH₂-CH₂-F, -0-CH₂-F, -0-CH₂-CH₂-F, -CH₂-OH, -CH₂-CH₂-OH, CF₃, -CH₂-OMe, - CH₂-CH₂-OMe, -CH₂-OEt, -CH₂-CH₂-OEt, -0-COMe, -O-COEt, -Q-COCF₃, -Q-COCF₃, fluorine, chlorine or bromine;
c) compounds of formula 1d 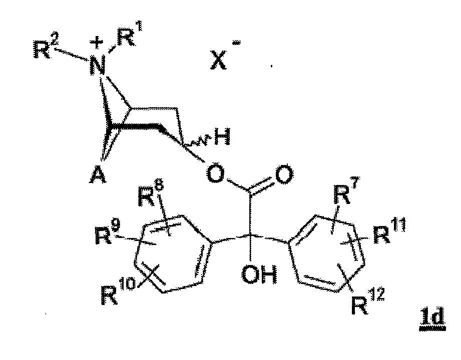 wherein
   A, **X⁻**, R¹ and R² may have the meanings as mentioned hereinbefore and wherein R⁷, R⁸, R⁹, R¹⁰, R¹¹ and R¹², which may be identical or different, denote hydrogen, methyl, ethyl, methyloxy, ethyloxy, hydroxy, fluorine, chlorine, bromine, CN, CF₃ or NO₂, with the proviso that at least one of the groups R⁷, R⁸, R⁹, R¹⁰, R¹¹ and R¹² is not hydrogen,
d) compounds of formula 1e 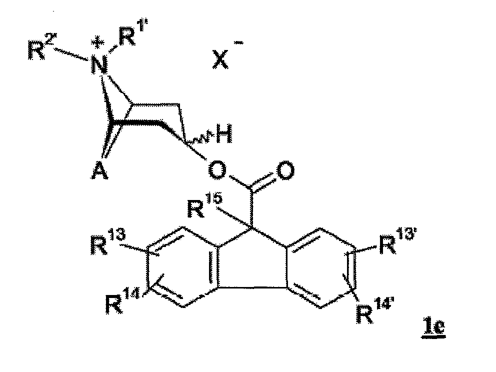 wherein A and **X⁻** may have the meanings as mentioned hereinbefore, and wherein R¹⁵ denotes hydrogen, hydroxy, methyl, ethyl, -CF₃, CHF₂ or fluorine;
   R^{1'} and R^{2'} which may be identical or different denote C₁-C₅-alkyl which may optionally be substituted by C₃-C₆-cycloalkyl, hydroxy or halogen, or
   R^{1'} and R2' together denote a -C₃-C₅-alkylene-bridge;
   R¹³, R¹⁴, R^{13'} and R^{14'} which may be identical or different denote hydrogen, -C₁-C₄-alkyl, -C₁-C₄-alkyloxy, hydroxy, -CF₃, -CHF₂, CN, NO₂ or halogen,
e) compounds of formula 1f 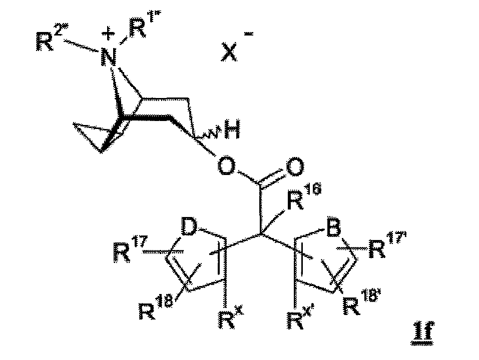 wherein **X⁻** may have the meanings as mentioned hereinbefore, and wherein
   D and B which may be identical or different, preferably identical, denote -O, -S, -NH, - CH₂, -CH=CH, or -N(C₁-C₄-alkyl)-;
   R¹⁶ denotes hydrogen, hydroxy, -C₁-C₄-alkyl, -C₁-C₄-alkyloxy, -C₁-C₄ - alkylene-Halogen, -O-C₁-C₄ alkylene-halogen, -C₁-C₄-alkylene-OH, -CF₃, CHF₂, -C₁-C₄-alkylene-C₁-C₄ alkyloxy, -O- COC₁-C₄-alkyl, -O-COC₁-C₄ -alkylene-halogen, -C₁-C₄-alkylene-C₃-C₆-cycloalkyl, -O-COCF₃ or halogen;
   R^{1"} and R^{2"} which may be identical or different, denote -C₁-C₅-alkyl, which may optionally be substituted by -C₃-C₆-cycloalkyl, hydroxy or halogen, or
   R^{1"} and R^{2"} together denote a -C₃-C₅-alkylene bridge;
   R¹⁷, R¹⁸, R^{17'} and R^{18'}, which may be identical or different, denote hydrogen, C₁-C₄-alkyl, C₁-C₄-alkyloxy, hydroxy, -CF₃, -CHF₂, CN, NO₂ or halogen;
   R^{x} and R^{x'} which may be identical or different, denote hydrogen, C₁-C₄-alkyl, C₁-C₄-alkyloxy, hydroxy, -CF₃, -CHF₂, CN, NO₂ or halogen or
   R^{x} and R^{x'} together denote a single bond or a bridging group selected from among the bridges -O, -S, -NH, -CH₂, -CH₂-CH₂-, -N(C₁-C₄-alkyl), -CH(C₁-C₄-alkyl)- and -C(C₁-C₄-alkyl)₂, and
f) compounds of formula 1g 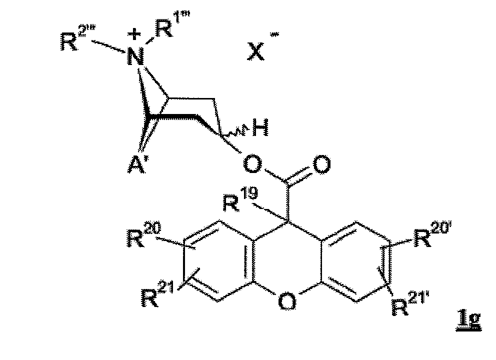 wherein **X⁻** may have the meanings as mentioned hereinbefore, and wherein A' denotes a double-bonded group selected from among 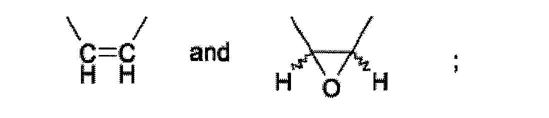 R¹⁹ denotes hydroxy, methyl, hydroxymethyl, ethyl, -CF₃, CHF₂ or fluorine;
   R^{1'''} and R^{2'''} which may be identical or different denote C₁-C₅-alkyl which may optionally be substituted by C₃-C₆-cycloalkyl, hydroxy or halogen, or
   R^{1'''} and R^{2'''} together denote a -C₃-C₅-alkylene-bridge;
   R²⁰, R²¹, R^{20'} and R^{21'} which may be identical or different denote hydrogen, -C₁-C₄-alkyl, -C₁-C₄-alkyloxy, hydroxy, -CF₃, -CHF₂, CN, NO₂ or halogen.

The compounds of formula 1c are known in the art (WO 02/32899).

In a preferred embodiment of the invention the method comprises administration of compounds of formula 1c, wherein
**X⁻** denotes bromide;
R¹ and R² which may be identical or different denote a group selected from methyl and ethyl, preferably methyl;
R³, R⁴, R⁵ and R⁶, which may be identical or different, denote hydrogen, methyl, methyloxy, chlorine or fluorine;
R⁷ denotes hydrogen, methyl or fluorine, optionally together with a pharmaceutically acceptable excipient.

Of particular importance are compounds of general formula 1c, wherein A denotes a double-bonded group selected from among

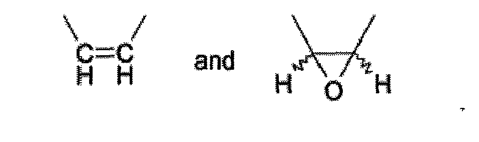

The compounds of formula 1c, may optionally be administered in the form of the individual optical isomers, mixtures of the individual enantiomers or racemates thereof.

Of particular importance within a method according to the invention are the following compounds of formula 1c:
tropenol 2,2-diphenylpropionic acid ester methobromide,
scopine 2,2-diphenylpropionic acid ester methobromide,
scopine 2-fluoro-2,2-diphenylacetic acid ester methobromide and
tropenol 2-fluoro-2,2-diphenylacetic acid ester methobromide.

The compounds of formula 1d are known in the art (WO 02/32898).

In a preferred embodiment of the invention the method comprises administration of compounds of formula 1d, wherein
A denotes a double-bonded group selected from among 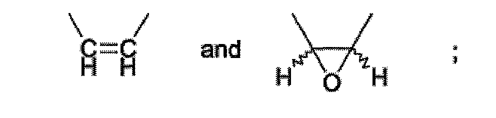 **X⁻** denotes bromide;
R¹ and R² which may be identical or different denote methyl or ethyl, preferably methyl;
R7, R⁸, R⁹, R¹⁰, R¹¹ and R¹², which may be identical or different, denote hydrogen, fluorine, chlorine or bromine, preferably fluorine with the proviso that at least one of the groups R⁷, R⁸, R⁹, R¹⁰, R¹¹ and R¹² not hydrogen, optionally together with a pharmaceutically acceptable excipient.

Of particular importance within the method according to the invention are the following compounds of formula 1d:
tropenol 3,3',4,4'-tetrafluorobenzilic acid ester methobromide,
scopine 3,3',4,4'-tetrafluorobenzilic acid ester methobromide,
scopine 4,4'-difluorobenzilic acid ester methobromide,
tropenol 4,4'-difluorobenzilic acid ester methobromide,
scopine 3,3'-difluorobenzilic acid ester methobromide, and
tropenol 3,3'-difluorobenzilic acid ester methobromide.

The pharmaceutical compositions according to the invention may contain the compounds of formula 1d optionally in the form of the individual optical isomers, mixtures of the individual enantiomers or racemates thereof.

The compounds of formula 1e are known in the art (WO 03/064419).

In a preferred embodiment of the invention the method comprises administration of compounds of formula 1e, wherein
A denotes a double-bonded group selected from among 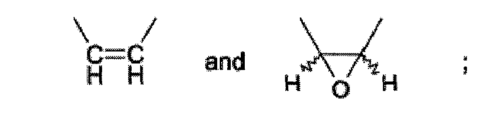 **X⁻** denotes an anion selected from among chloride, bromide and methanesulphonate, preferably bromide;
R¹⁵ denotes hydroxy, methyl or fluorine, preferably methyl or hydroxy;
R^{1'} and R^{2'} which may be identical or different represent methyl or ethyl, preferably methyl;
R¹³, R¹⁴, R^{13'} and R^{14'} which may be identical or different represent hydrogen, -CF₃, - CHF₂ or fluorine, preferably hydrogen or fluorine, optionally together with a pharmaceutically acceptable excipient.

In another preferred embodiment of the invention the method comprises administration of compounds of formula 1e, wherein
A denotes a double-bonded group selected from among 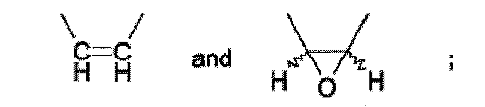 **X⁻** denotes bromide;
R¹⁵ denotes hydroxy or methyl, preferably methyl;
R^{1'} and R^{2'} which may be identical or different represent methyl or ethyl, preferably methyl;
R¹³, R¹⁴, R^{13'} and R^{14'} which may be identical or different represent hydrogen or fluorine, optionally together with a pharmaceutically acceptable excipient.

Of particular importance within the method according to the invention are the following compounds of formula 1e:
tropenol 9-hydroxy-fluorene-9-carboxylate methobromide ;
tropenol 9-fluoro-fluorene-9-carboxylate methobromide;
scopine 9-hydroxy-fluorene-9-carboxylate methobromide;
scopine 9-fluoro-fluorene-9-carboxylate methobromide;
tropenol 9-methyl-fluorene-9-carboxylate methobromide;
scopine 9-methyl-fluorene-9-carboxylate methobromide.

The pharmaceutical compositions according to the invention may contain the compounds of formula 1e optionally in the form of the individual optical isomers, mixtures of the individual enantiomers or racemates thereof.

The compounds of formula 1f are known in the art (WO 03/064418).

In another preferred embodiment of the invention the method comprises administration of compounds of formula 1f wherein
**X⁻** denotes chloride, bromide, or methanesulphonate, preferably bromide;
D and B which may be identical or different, preferably identical, denote -O, -S, -NH or -CH=CH-;
R¹⁶ denotes hydrogen, hydroxy, -C₁-C₄-alkyl, -C₁-C₄ alkyloxy, -CF₃, -CHF₂, fluorine, chlorine or bromine;
R^{1"} and R^{2"} which may be identical or different, denote C₁-C₄-alky, which may optionally be substituted by hydroxy, fluorine, chlorine or bromine, or
R^{1"} and R^{2"} together denote a -C₃-C₄-alkylene-bridge;
R¹⁷, R¹⁸, R^{17'} and R^{18'}, which may be identical or different, denote hydrogen, C₁-C₄-alkyl, C₁-C₄-alkyloxy, hydroxy, -CF₃, -CHF₂, CN, NO₂, fluorine, chlorine or bromine; R^{x} and R^{x'} which may be identical or different, denote hydrogen, C₁-C₄-alkyl, C₁-C₄-alkyloxy, hydroxy, -CF₃, -CHF₂, CN, NO₂, fluorine, chlorine or bromine or R^{x} and R^{x'} together denote a single bond or a bridging group selected from among the bridges -O, -S, -NH- and -CH₂-, optionally together with a pharmaceutically acceptable excipient.

In another preferred embodiment of the invention the method comprises administration of compounds of formula 1f, wherein
**X⁻** denotes chloride, bromide, or methanesulphonate, preferably bromide;
D and B which may be identical or different, preferably identical, denote -S or - CH=CH-;
R¹⁶ denotes hydrogen, hydroxy or methyl;
R^{1"} and R^{2"} which may be identical or different, denote methyl or ethyl;
R¹⁷, R¹⁸, R^{17'} and R^{18'}, which may be identical or different, denote hydrogen, -CF₃ or fluorine, preferably hydrogen;
R^{x} and R^{x'} which may be identical or different, denote hydrogen, -CF₃ or fluorine, preferably hydrogen or
R^{x} and R^{x'} together denote a single bond or the bridging group -O-, optionally together with a pharmaceutically acceptable excipient.

In another preferred embodiment of the invention the method comprises administration of compounds of formula lf wherein
**X⁻** denotes bromide;
D and B denote -CH=CH-;
R¹⁶ denotes hydrogen, hydroxy or methyl;
R^{1"} and R^{2"} denote methyl;
R¹⁷, R¹⁸, R^{17'} and R^{18'}, which may be identical or different, denote hydrogen or fluorine, preferably hydrogen;
R^{x} and R^{x'} which may be identical or different, denote hydrogen or fluorine, preferably hydrogen or
R^{x} and R^{x'} together denote a single bond or the bridging group -O-, optionally together with a pharmaceutically acceptable excipient.

Of particular importance within the method according to the invention are the following compounds of formula 1f:
cyclopropyltropine benzilate methobromide;
cyclopropyltropine 2,2-diphenylpropionate methobromide;
cyclopropyltropine 9-hydroxy-xanthene-9-carboxylate methobromide;
cyclopropyltropine 9-methyl-fluorene-9-carboxylate methobromide; cyclopropyltropine 9-methyl-xanthene-9-carboxylate methobromide; cyclopropyltropine 9-hydroxy-fluorene-9-carboxylate methobromide; cyclopropyltropine methyl 4,4'-difluorobenzilate methobromide.

The pharmaceutical compositions according to the invention may contain the compounds of formula 1f optionally in the form of the individual optical isomers, mixtures of the individual enantiomers or racemates thereof.

The compounds of formula 1g are known in the art (WO 03/064417).

In another preferred embodiment of the invention the method comprises administration of compounds of formula 1g wherein
A' denotes a double-bonded group selected from among 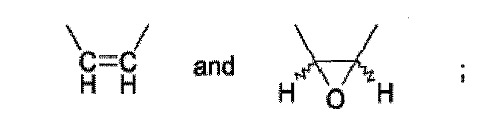 **X⁻** denotes chloride, bromide or methanesulphonate, preferably bromide;
R¹⁹ denotes hydroxy or methyl;
R^{1'''} and R^{2'''} which may be identical or different represent methyl or ethyl, preferably methyl;
R²⁰, R²¹, R^{20'} and R^{21'} which may be identical or different represent hydrogen, -CF₃,-CHF₂ or fluorine, preferably hydrogen or fluorine, optionally together with a pharmaceutically acceptable excipient.

In another preferred embodiment of the invention the method comprises administration of compounds of formula 1g wherein
A' denotes a double-bonded group selected from among 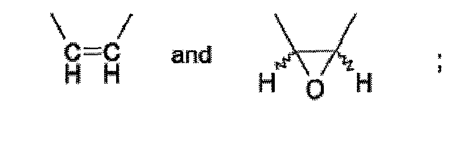 **X⁻** denotes bromide;
R¹⁹ denotes hydroxy or methyl, preferably methyl;
R^{1'''} and R^{2'''} which may be identical or different represent methyl or ethyl, preferably methyl;
R³, R⁴, R^{3'} and R^{4'} which may be identical or different represent hydrogen or fluorine, optionally together with a pharmaceutically acceptable excipient.

Of particular importance within the method according to the invention are the following compounds of formula 1g:
tropenol 9-hydroxy-xanthene-9-carboxylate methobromide;
scopine 9-hydroxy-xanthene-9-carboxylate methobromide;
tropenol 9-methyl-xanthene-9-carboxylate methobromide;
scopine 9-methyl-xanthene-9-carboxylate methobromide;
tropenol 9-ethyl-xanthene-9-carboxylate methobromide;
tropenol 9-difluoromethyl-xanthene-9-carboxylate methobromide;
scopine 9-hydroxymethyl-xanthene-9-carboxylate methobromide.

The pharmaceutical compositions according to the invention may contain the compounds of formula 1g optionally in the form of the individual optical isomers, mixtures of the individual enantiomers or racemates thereof.

The alkyl groups used, unless otherwise stated, are branched and unbranched alkyl groups having 1 to 5 carbon atoms. Examples include: methyl, ethyl, propyl or butyl. The groups methyl, ethyl, propyl or butyl may optionally also be referred to by the abbreviations Me, Et, Prop or Bu. Unless otherwise stated, the definitions propyl and butyl also include all possible isomeric forms of the groups in question. Thus, for example, propyl includes n- propyl and iso-propyl, butyl includes iso-butyl, sec. butyl and tert. -butyl, etc.

The cycloalkyl groups used, unless otherwise stated, are alicyclic groups with 3 to 6 carbon atoms. These are the cyclopropyl, cyclobutyl, cyclopentyl and cyclohexyl groups.
According to the invention cyclopropyl is of particular importance within the scope of the present invention.

The alkylene groups used, unless otherwise stated, are branched and unbranched double- bonded alkyl bridges with 1 to 5 carbon atoms. Examples include: methylene, ethylene, propylene or butylene.

The alkylene-halogen groups used, unless otherwise stated, are branched and unbranched double-bonded alkyl bridges with 1 to 4 carbon atoms which may be mono-, di- or trisubstituted, preferably disubstituted, by a halogen. Accordingly, unless otherwise stated, the term alkylene-OH groups denotes branched and unbranched double-bonded alkyl bridges with 1 to 4 carbon atoms which may be mono-, di- or trisubstituted, preferably monosubstituted, by a hydroxy.

The alkyloxy groups used, unless otherwise stated, are branched and unbranched alkyl groups with 1 to 5 carbon atoms which are linked via an oxygen atom. The following may be mentioned, for example: methyloxy, ethyloxy, propyloxy or butyloxy. The groups methyloxy, ethyloxy, propyloxy or butyloxy may optionally also be referred to by the abbreviations MeO, EtO, PropO or BuO. Unless otherwise stated, the definitions propyloxy and butyloxy also include all possible isomeric forms of the groups in question. Thus, for example, propyloxy includes n-propyloxy and iso-propyloxy, butyloxy includes iso-butyloxy, sec. butyloxy and tert. -butyloxy, etc. The word alkoxy may also possibly be used within the scope of the present invention instead of the word alkyloxy. The groups methyloxy, ethyloxy, propyloxy or butyloxy may optionally also be referred to as methoxy, ethoxy, propoxy or butoxy.

The alkylene-alkyloxy groups used, unless otherwise stated, are branched and unbranched double-bonded alkyl bridges with 1 to 5 carbon atoms which may be mono-, di- or trisubstituted, preferably monosubstituted, by an alkyloxy group.

The -O-CO-alkyl groups used, unless otherwise stated, are branched and unbranched alkyl groups with 1 to 4 carbon atoms which are bonded via an ester group. The alkyl groups are bonded directly to the carbonylcarbon of the ester group. The term -O-CO-alkyl-halogen group should be understood analogously. The group -O-CO-CF₃ denotes trifluoroacetate.

Within the scope of the present invention halogen denotes fluorine, chlorine, bromine or iodine. Unless otherwise stated, fluorine and bromine are the preferred halogens. The group CO denotes a carbonyl group.

The inhalation device according to the invention comprises the compounds of formula 1 preferably in admixture with a pharmaceutically acceptable excipient to form a powder mixture. The following pharmaceutically acceptable excipients may be used to prepare these inhalable powder mixtures according to the invention: monosaccharides (e.g. glucose or arabinose), disaccharides (e.g. lactose, saccharose, maltose, trehalose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose and trehalose are the particularly preferred excipients, while lactose, preferably in form of its monohydrate is most particularly preferred.

The compounds of formula 1 may be used in the form of their racemates, enantiomers or mixtures thereof. The separation of enantiomers from the racemates may be carried out using methods known in the art (e.g. by chromatography on chiral phases, etc.).

Optionally, the inhalation device according to the invention contains plural of doses of a medicament in powder form that contains, beside one compound of formula 1, another active ingredient.

Preferably the additional active ingredient is a beta₂ agonists 2 which is selected from the group consisting of albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, rimiterol, ritodrine, salmeterol, salmefamol, soterenot, sulphonterol, tiaramide, terbutaline, tolubuterol, CHF-1035, HOKU-81, KUL-1248, 3-(4-{6-[2-Hydroxy-2-(4-hydroxy-3- hydroxymethyl-phenyl)-ethylamino]-hexyloxy}-butyl)-benzenesulfoneamide, 5-[2-(5,6- Diethyl-indan-2-ylamino)-1-hydroxy-ethyl]-8-hydroxy-1H-quinolin-2-one , 4-hydroxy-7- [2- { [2- { [3 -(2-phenylethoxy)propyl] sulphonyl} ethyl] -amino} ethyl] -2(3H)-benzothiazolone , 1 -(2-fluoro-4-hydroxyphenyl)-2-[4-(1-benzimidazolyl)-2-methyl-2-butylamino]ethanol, 1 - [3 -(4-methoxybenzyl-amino)-4-hydroxyphenyl] -2- [4-( 1 -benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4- benzoxazin-8-yl]-2-[3-(4-methoxyphenyl)-2-methyl-2-propylamino]ethanol, 1 -[2H-5- hydroxy-3 -0X0-4H- 1 ,4-benzoxazin-8-yl] -2- [3 -(4-n-butyloxyphenyl)-2-methyl-2-propylamino]ethanol, 1 - [2H-5-hydroxy-3 -oxo-4H- 1 ,4-benzoxazin-8-yl] -2- {4- [3 - (4- methoxyphenyl)-1,2,4-triazol-3-yl]-2-methyl-2-butylamino} ethanol , 5-hydroxy-8-(1-hydroxy-2-isopropylaminobutyl)-2H-l,4-benzoxazin-3-(4H)-one, 1-(4-amino-3-chloro-5-trifluormethylphenyl)-2-tert-butylamino)ethanol and 1 -(4-ethoxycarbonylamino-3-cyano- 5-fluorophenyl)-2-(tert.-butylamino)ethanol, optionally in the form of the racemates, the enantiomers, the diastereomers and optionally the pharmacologically acceptable acid addition salts and the hydrates thereof.

According to the instant invention more preferred beta₂ agonists 2 are selected from the group consisting of bambuterol, bitolterol, carbuterol, clenbuterol, fenoterol, formoterol, hexoprenaline, ibuterol, pirbuterol, procaterol, reproterol, salmeterol, sulphonterol, terbutaline, tolubuterol, 3-(4- {6-[2-Hydroxy-2-(4-hydroxy-3-hydroxymethyl-phenyl)- ethylamino]-hexyloxy} -butyl)-benzenesulfoneamide, 5-[2-(5,6-Diethyl-indan-2-ylamino)-1-hydroxy-ethyl]-8-hydroxy-1H-quinolin-2-one, 4-hydroxy-7-[2-{[2-{[3-(2- phenylethoxy)propyl]sulphonyl} ethyl]-amino} ethyl]-2(3H)-benzothiazolone, 1-(2-fluoro- 4-hydoxyphenyl)-2-[4-(1 -benzimidazolyl)-2-methyl-2-butylamino]ethanol, 1 -[3-(4- methoxybenzyl-amino)-4-hydroxyphenyl] -2- [4-(1 - benzimidazolyl)-2-methyl-2- butylamino]ethanol ,1-[2H-5-hydroxy-3-oxo-4H-1,4-benzoxazin-8-yl]-2-[3-(4-N,N- dimethylaminophenyl)-2-methyl-2-propylamino]ethanol , 1-[2H-5-hydroxy-3-oxo-4H-1,4- benzoxazin-8-yl]-2-[3-(4-methoxyphenyl)-2-methyl-2-propylamino]ethanol, 1 -[2H-5- hydroxy-3 -0X0-4H- 1 ,4-benzoxazin-8-yl] -2- [3 -(4-n-butyloxyphenyl)-2-methyl-2- propylamino]ethanol , 1 - [2H-5-hydroxy-3 -oxo-4H- 1 ,4-benzoxazin-8-yl] -2- {4- [3 -(4- methoxyphenyl)-1,2,4-triazol-3-yl]-2-methyl-2-butylamino}ethanol, 5-hydroxy-8-(1- hydroxy-2-isopropylaminobutyl)-2H-1,4-benzoxazin-3-(4H)-one,1-(4-amino-3-chloro-5- trifluormethylphenyl)-2-tert.-butylamino)ethanol and 1 -(4-ethoxycarbonylamino-3-cyano- 5-fluorophenyl)-2-(tert-butylamino)ethanol, optionally in the form of the racemates, the enantiomers, the diastereomers and optionally the pharmacologically acceptable acid addition salts and the hydrates thereof.

More preferably, the betamimetics 2 used as within the compositions according to the invention are selected from among fenoterol, formoterol, salmeterol, 3-(4-{6-[2-Hydroxy- 2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1H-quinolin-2-one, 1 -[3-(4-methoxybenzyl-amino)-4-hydroxyphenyl]-2-[4-(1-benzimidazolyl)-2-methyl-2-butylamino]ethanol 1-[2H-5-hydroxy-3-oxo-4H-1,4-benzoxazin-8-yl]-2-[3-(4-N,N-dimethylaminophenyl)-2-methyl-2-propylamino]ethanol, 1 - [2H-5-hydroxy-3-oxo-4H-1,4-benzoxazin-8-yl]-2-[3-(4-methoxyphenyl)-2-methyl-2-propylamino] ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl] -2- {4- [3 -(4-methoxyphenyl)- 1 ,2,4-triazol-3 -yl] -2-methyl-2-butylamino}ethanol, optionally in the form of the racemates, the enantiomers, the diastereomers and optionally the pharmacologically acceptable acid addition salts thereof, and the hydrates thereof. Of the betamimetics mentioned above the compounds formoterol, salmeterol, 3-(4-{6-[2-Hydroxy-2-(4-hydroxy-3-hydroxymethylphenyl)-ethylamino]- hexyloxy}-butyl)-benzenesulfoneamide, and 5-[2-(5,6-Diethyl-indan-2-ylamino)-1- hydroxy-ethyl]-8-hydroxy-1H-quinolin-2-one are particularly preferred, optionally in the form of the racemates, the enantiomers, the diastereomers and optionally the pharmacologically acceptable acid addition salts thereof, and the hydrates thereof. Of the betamimetics mentioned above the compounds formoterol and salmeterol are particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the betamimetics 2 according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4- difluorophenyl)salicylic acid or maleic acid. If desired, mixtures of the abovementioned acids may also be used to prepare the salts 2.

According to the invention, the salts of the betamimetics 2 selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of 2 in the case of salmeterol selected from among the hydrochloride, sulphate, 4-phenylcinnamate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of 2 in the case of formoterol selected from the hydrochloride, sulphate and fumarate, of which the hydrochloride and fumarate are particularly preferred, such as formoterol fumarate.

Salts of salmeterol, formoterol, 3-(4-{6-[2-Hydroxy-2-(4-hydroxy-3-hydroxymethylphenyl)-ethylamino]-hexyloxy} -butyl)-benzenesulfoneamide, and 5-[2-(5,6-Diethyl-indan- 2-ylamino)-1-hydroxy-ethyl]-8-hydroxy-1H-quinolin-2-one , are preferably used as the betamimetics 2 according to the invention. Of particular importance are salmeterol and formoterol salts. Any reference to the term betamimetics 2 also includes a reference to the relevant enantiomers or mixtures thereof. In the pharmaceutical compositions according to the invention, the compounds 2 may be present in the form of their racemates, enantiomers or mixtures thereof. The separation of the enantiomers from the racemates may be carried out using methods known in the art (e.g. by chromatography on chiral phases, etc.) If the compounds 2 are used in the form of their enantiomers, it is particularly preferable to use the enantiomers in the R configuration at the C-OH group.

Optionally, the inhalation device according to the invention contains plural of doses of a medicament in powder form that contains beside one compound of formula 1 a steroid 3 as another active ingredient.

In such medicament combinations the steroid 3 is preferably selected from among prednisolone, prednisone , butixocortpropionate, RPR- 106541, flunisolide , beclomethasone , triamcinolone , budesonide , fluticasone, mometasone , ciclesonide , rofleponide , ST- 126, dexamethasone , (S)-fluoromethyl 6α,9α-difluoro-17α-[(2-furanylcarbonyl)oxy] - 11 [beta]-hydroxy- 16α-methyl-3 -oxo-androsta- 1 ,4-diene- 17β-carbothionate , (S)-(2-oxo-tetrahydro-furan-3S-yl)6α,9α-difluoro-11 β-hydroxy- 16α-methyl-3 -oxo- 17α-propionyloxy-androsta- 1 ,4-diene- 17β-carbothionate, and etiprednol- dichloroacetate (BNP- 166), optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising flunisolide , beclomethasone , triamcinolone, budesonide , fluticasone, mometasone , ciclesonide , rofleponide, ST- 126, dexamethasone , (S)-fluoromethyl 6α,9α-difluoro- 1 Ia- [(2-furanylcarbonyl)oxy]- 11 β-hydroxy-16α-methyl-3 -oxo-androsta-1,4-diene-17β-carbothionate, (S)-(2-oxo-tetrahydro-furan-3S-yl)6α,9α-difluoro-11β- hydroxy- 16α-methyl-3 -oxo- 17α-propionyloxy-androsta- 1 ,4-diene- 17β-carbothionate, and etiprednol-dichloroacetate , optionally in the form of the racemates, enantiomers or diastereomers thereof and optionally in the form of the salts and derivatives thereof, the solvates and/or hydrates thereof.

In particularly preferred medicament combinations the steroid 3 is selected from the group comprising budesonide , fluticasone , mometasone , ciclesonide , (S)-fluoromethyl 6α,9α-difluoro- 1 Ia- [(2-furanylcarbonyl)oxy] - 11 β-hydroxy- 16α-methyl-3 -oxo-androsta- 1 ,A- diene-17β-carbothionate , and etiprednol-dichloroacetate , optionally in the form of the racemates, enantiomers or diastereomers thereof and optionally in the form of the salts and derivatives thereof, the solvates and/or hydrates thereof.

Any reference to steroids 3 includes a reference to any salts or derivatives, hydrates or solvates thereof which may exist. Examples of possible salts and derivatives of the steroids 3 may be: alkali metal salts, such as for example sodium or potassium salts, sulphobenzoates, phosphates, isonicotinates, acetates, propionates, dihydrogen phosphates, palmitates, pivalates or furcates.

Optionally, the inhalation device according to the invention contains plural of doses of a medicament on powder form that contains beside one compound of formula 1 additionally both, one of the betamimetics 2 mentioned hereinbefore and one of the steroids 3 mentioned hereinbefore.

According to one aspect, there is provided an inhalation device according to the invention, wherein each blister contains a pharmaceutical composition in powder form wherein the pharmaceutical composition comprises one or more, preferably one, compound of formula 1.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipients mentioned above. These finer excipients are also selected from the group of possible excipients listed hereinbefore. Finally, in order to prepare the inhalable powders according to the invention, micronised active substance I-, and optionally 2 and/or 3, preferably with an average particle size of 0.5 to 10µm, more preferably from 1 to 6µm, is added to the excipient mixture. Processes for producing the inhalable powders according to the invention by grinding and micronising and finally mixing the ingredients together are known from the prior art.

For the methods of preparing the pharmaceutical compositions in powder form reference may be made to the disclosure of WO 02/30390, WO 03/017970, or WO 03/017979 for example. The disclosure of WO 02/30390, WO 03/017970, and WO 03/017979 is herby incorporated by reference into the instant patent application in its entirety.

As an example, the pharmaceutical compositions according to the invention may be obtained by the method described below.

First, the excipient and the active substance are placed in a suitable mixing container. The active substance used has an average particle size of 0.5 to 10 µm, preferably 1 to 6 µm, most preferably 2 to 5 µm. The excipient and the active substance are preferably added using a sieve or a granulating sieve with a mesh size of 0.1 to 2 mm, preferably 0.3 to 1 mm, most preferably 0.3 to 0.6 mm. Preferably, the excipient is put in first and then the active substance is added to the mixing container. During this mixing process the two components are preferably added in batches. It is particularly preferred to sieve in the two components in alternate layers. The mixing of the excipient with the active substance may take place while the two components are still being added. Preferably, however, mixing is only done once the two components have been sieved in layer by layer.

If after being chemically prepared the active substance used in the process described above is not already obtainable in a crystalline form with the particle sizes mentioned earlier, it can be ground up into the particle sizes which conform to the abovementioned parameters (so-called micronising).
Many modifications and variations of the invention falling within the terms of the following claims will be apparent to those skilled in the art and the foregoing description should be regarded as a description of the preferred embodiments of the invention only.

## Claims

1. An inhaler comprising a housing to receive a strip of sealed blisters each containing a dose of medicament, means to move a strip received within the housing such that each blister is sequentially aligned with a blister opening member for opening said aligned blister to facilitate inhalation of said dose and, means for re-sealing opened blisters previously aligned with the blister opening member by applying adhesive tape to the strip.

2. An inhaler according to claim 1, wherein said means for re-sealing opened blisters is configured to re-seal opened blisters in response to operation of the means for moving the strip.

3. An inhaler according to claim 1 or claim 2, wherein the means for re-sealing opened blisters includes a shaft in the housing to receive a reel of adhesive tape such that it can be unwound from the reel and adhered to a surface of the strip to re-seal opened blisters.

4. An inhaler according to claim 3, wherein the shaft is configured to receive a reel of adhesive tape freely mounted for rotation about the shaft.

5. An inhaler according to claim 3 or claim 4, wherein said means for re-sealing opened blisters further comprises a guide element parallel to but spaced from the shaft around which adhesive tape is passed as it is unwound from a reel and before being adhered to the surface of a strip.

6. An inhaler according to claim 5, wherein the guide element is fixed and the adhesive tape slides over it as it is unwound from a reel.

7. An inhaler according to claim 3 or 4, wherein the shaft has an axis that lies parallel to but spaced from the surface of a strip received in the housing, said shaft being movably mounted to the housing.

8. An inhaler according to claim 7, wherein the shaft is mounted such that, when adhesive tape is pulled from a reel received on the shaft, the axis of the shaft is drawn closer to said surface.

9. An inhaler according to claim 7 or claim 8, comprising biasing means to bias the shaft in a direction towards the surface of a strip.

10. An inhaler according to any of claims 7 to 9, wherein the shaft extends from one end of an arm pivotally mounted to the housing at its opposite end.

11. An inhaler according to any of claims 3 to 10, including guide slots in the housing to slideably receive the ends of the shaft.

12. An inhaler according to claim 11, wherein the guide slots are configured such that the ends of the shaft slide in the slots such that the shaft moves towards the surface of a strip when adhesive tape is pulled from a reel.

13. An inhaler according to any preceding claim, including a reel of adhesive tape received in the housing.

14. An inhaler according to claim 13, including a coiled strip of blisters received within the housing.

15. An inhaler according to claim 14, wherein the axis about which the reel is mounted for rotatation to unwind the adhesive tape is substantially parallel to the axis about which the coiled strip rotates to uncoil the strip during operation of the means for moving the strip within the housing.

16. An inhaler according to claim 14 or claim 15, wherein a leading end of the adhesive tape is unwound from the reel and adhered to a surface of the strip.

17. An inhaler according to any of claims 13 to 16, wherein the reel of adhesive tape has one adhesive side, said reel being wound such that said adhesive side faces inward towards the axis of the shaft.

18. An inhaler according to any of claims 13 to 17, wherein the reel of adhesive tape has one adhesive side, said reel being wound such that said adhesive side faces outward away from the axis of the shaft.

19. A method of assembling an inhaler comprising a housing to receive a strip of sealed blisters each containing a dose of medicament, the method including the step of placing a coiled strip of blisters in the housing together with a reel of adhesive tape, a leading end of said adhesive tape being unwound from the reel and adhered to the surface of the strip.
